(19) Europäisches Patentamt
European Patent Office
Office européen des brevets

(11) EP 0 828 715 B1

(12) **EUROPÄISCHE PATENTSCHRIFT**

(45) Veröffentlichungstag und Bekanntmachung des Hinweises auf die Patenterteilung:
**16.08.2001 Patentblatt 2001/33**

(21) Anmeldenummer: **96916138.9**

(22) Anmeldetag: **20.05.1996**

(51) Int Cl.[7]: **C07D 215/56**, A61K 31/47, A61K 31/495, C07D 498/16

(86) Internationale Anmeldenummer:
**PCT/EP96/02171**

(87) Internationale Veröffentlichungsnummer:
**WO 96/38417 (05.12.1996 Gazette 1996/53)**

(54) **N-OXIDE ALS ANTIBAKTERIELLE MITTEL**

N-OXIDES AS ANTIBACTERIAL AGENTS

N-OXIDES UTILISES COMME AGENTS ANTIBACTERIENS

(84) Benannte Vertragsstaaten:
**AT BE CH DE DK ES FI FR GB GR IE IT LI NL SE**

(30) Priorität: **31.05.1995 DE 19519822**

(43) Veröffentlichungstag der Anmeldung:
**18.03.1998 Patentblatt 1998/12**

(73) Patentinhaber: **BAYER AG**
**51368 Leverkusen (DE)**

(72) Erfinder:
- **RAST, Hubert**
  **D-51381 Leverkusen (DE)**
- **SCHEER, Martin**
  **D-42113 Wuppertal (DE)**
- **HALLENBACH, Werner**
  **D-40789 Monheim (DE)**

(56) Entgegenhaltungen:
**EP-A- 0 090 424**

- **JOURNAL OF MEDICINAL CHEMISTRY, Bd. 27, Nr. 9, 1984, WASHINGTON US, Seiten 1103-1108, XP002012275 M.P. WENTLAND ET AL.: "Novel amino-substituted 3-quinolinecarboxylic acid antibacterial agents"**
- **ANTIMICROB. AGENTS CHEMOTH., Bd. 33, Nr. 5, 1989, Seiten 762-766, XP000600481 R.A. VENEZIA ET AL.: "In vitro activities of amifloxacin and two of its metabolites" in der Anmeldung erwähnt**
- **CHEMICAL ABSTRACTS, vol. 114, no. 13, 1991 Columbus, Ohio, US; abstract no. 114663w, Y. NIWATA ET AL.: "In vitro and in vivo antibacterial activity of urinary metabolites of fleroxacin" Seite 22; XP002012278 & CHEMOTHERAPY , Bd. 38, Nr. suppl. 2, 1990, TOKYO, Seiten 672-675,**
- **CHEMICAL ABSTRACTS REGISTRY HANDBOOK - NUMBER SECTION., COLUMBUS US, XP002012276 & CHEMICAL ABSTRACTS, vol. 109, no. 25, 1988 Columbus, Ohio, US; abstract no. 221823s, F. COLLIGNON ET AL.: "In vitro models" Seite 8;**
- **CHEMICAL ABSTRACTS REGISTRY HANDBOOK - NUMBER SECTION., COLUMBUS US, XP002012277 & J. CHROMATOGR., Nr. 413, 1987, Seiten 199-206, G.R. GRANNEMAN ET AL.: "HPLC procedures for the determination of difloxacin and it metabolites"**
- **PATHOL. BIOL., Bd. 37 , Nr. 5, 1989, Seiten 406-410, XP000600482 J. GUIBERT ET AL.: "Antibacterial activity of pefloxacin in urine" in der Anmeldung erwähnt**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 828 715 B1

## Beschreibung

Die vorliegende Erfindung betrifft neue antibakterielle Mittel, insbesondere oral verabreichbare Mittel, auf Basis von N-Oxiden gesättigter stickstoffhaltiger Heterocyclen die durch Chinoloncarbonsäuren oder Naphthyridoncarbonsäuren substituiert sind sowie neue Wirkstoffe und ihre Herstellung.

N-Oxide gesättigter stickstoffhaltiger Heterocyclen, die durch Chinoloncarbonsäuren substituiert sind, sind als Metaboliten der ihnen zugrunde liegenden Wirkstoffe bekannt. Es ist allerdings auch bekannt, daß diese Metaboliten keine antibakterielle Wirkung besitzen (J. Guibert et. al. Pathol. Biol. 1989, 37(5), 406-10; Venezia et. al. Antimicrob. Agents. Chemot. 1989, 33(5), 762-6).

Es war ferner bekannt, daß die orale Verabreichung von antibakteriellen Mitteln auf der Basis von Chinolon- oder Naphthyridoncarbonsäuren über das Futter oder Trinkwasser von Tieren oft problematisch ist. Es kommt häufig zu Trinkwasser-oder Futterverweigerung und damit zu unzureichender Dosierung des Medikaments. Da die Medikierung über das Futter oder das Trinkwasser eine sehr einfache und für Tierarzt, Tierhalter und zu behandelndes Tier streßfreie Art der Verabreichung ist, besteht ein hoher Bedarf an oral zu verabreichenden Mitteln. Es ist wünschenswert, gerade die hoch wirksamen antibakteriellen Mittel aus der Chinoloncarbonsäurereihe wie z.B. Enrofloxacin zuverlässig über das Futter verabreichen zu können.

Es wurde gefunden:

1. Antibakterielle Mittel auf Basis von N-Oxiden gesättigter stickstoffhaltiger Heterocyclen, die durch Chinolon- oder Naphthyridoncarbonsäuren substituiert sind der Formel (I) oder (II)

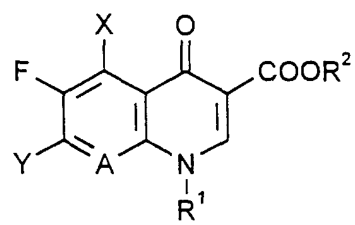

(I)

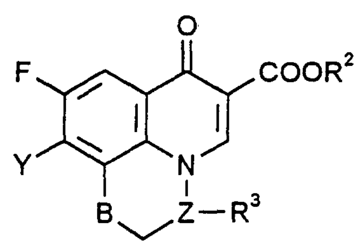

(II)

in welchen

X für Wasserstoff, Halogen, $C_{1-4}$-Alkyl, $C_{1-4}$-Alkoxy, $NH_2$ steht,

Y für Reste der Strukturen

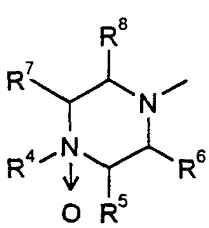 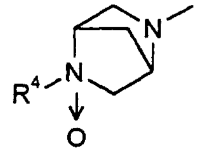 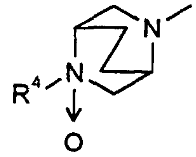 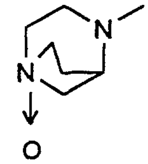

steht, worin

$R^4$ für gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

sowie

$R^1$ für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,

$R^2$ für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,

$R^3$ für Wasserstoff, Methyl oder Ethyl steht und

A für Stickstoff oder =CH-, =C(Halogen)-, = $C(OCH_3)$- =$C(CH_3)$-steht,

B für Sauerstoff, gegebenenfalls durch Methyl oder Phenyl substituiertes =NH sowie =$CH_2$ steht,

Z für =CH- oder =N- steht,

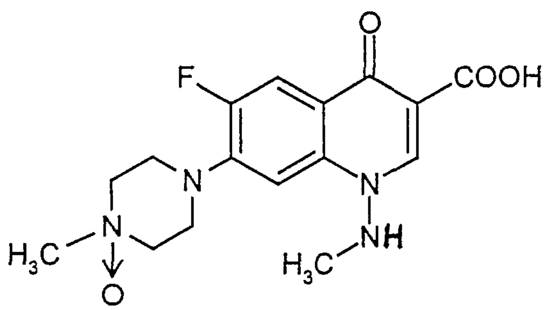

Amifloxacin-N-oxid,

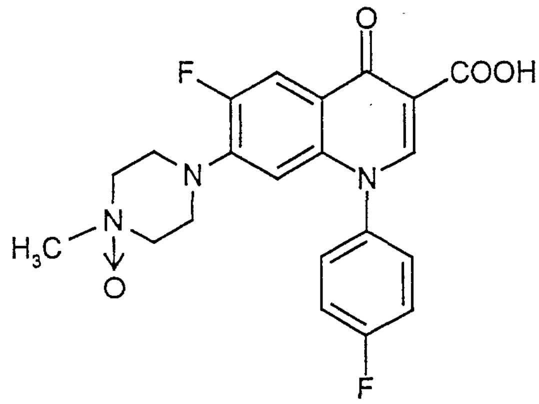

Difloxacin-N-oxid,

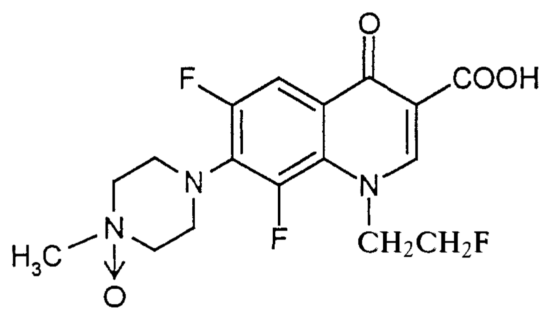

Fleroxacin-N-oxid und

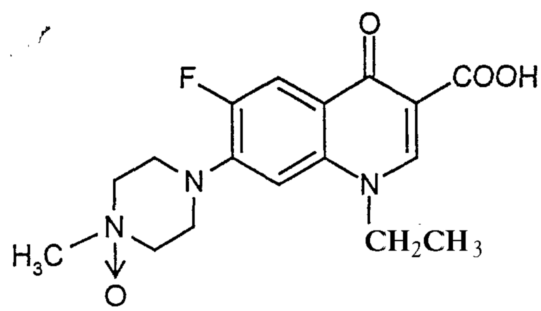

Pefloxacin-N-oxid ,

mit Ausnahme der folgenden Verbindungen: und deren pharmazeutisch verwendbare Hydrate, Säureadditionssalze und Salze mit Basen. Die Verbindungen der Formeln I und II können in Form ihrer Racemate oder in enantiomeren Formen vorliegen.

2. Über das Futter oder Trinkwasser von Tieren verabreichbare Mittel auf Basis von N-Oxiden gesättigter stickstoffhaltiger Heterocyclen, die durch Chinolon-oder Naphthyridoncarbonsäuren substituiert sind, der Formeln (I) oder (II) wie oben definiert, und deren pharmazeutisch verwendbare Hydrate, Säureadditionssalze und Salze mit Basen.

3. Neue N-Oxide gesättigter stickstoffhaltiger Heterocyclen, die durch Chinoloncarbonsäuren substituiert sind, der

Formel (Ia)

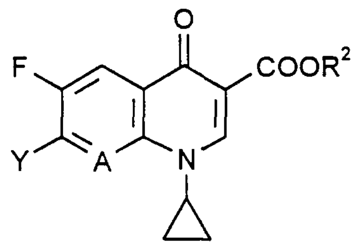

(Ia)

in welcher

Y für Reste der Strukturen

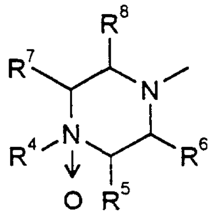 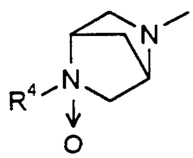 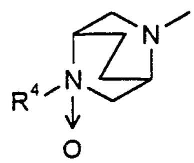 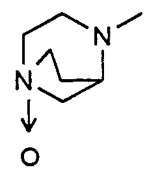

steht, worin

$R^4$ für gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

sowie

$R^2$ für Wasserstoff oder gegebenenfalls durch Methoxy substituiertes Alkyl mit 1 bis 4 Kohlenstoffatomen sowie Benzyl, 2-Oxopropyl, Phenacyl sowie Ethoxycarbonylmethyl steht,

A für =N-, =CH-, =C(Halogen)- oder =$C(OCH_3)$- steht,

sowie deren pharmazeutisch verwendbare Hydrate oder Säureadditionssalze oder Salze mit Basen.

Weiterhin wurde gefunden, daß man die Verbindungen der Formel (Ia) erhält, wenn man

a) die entsprechenden Verbindungen (III)

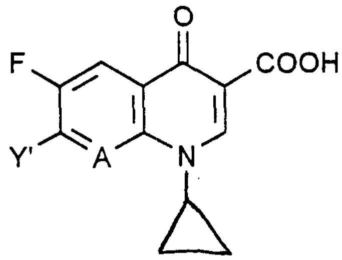

(III)

in welcher

Y' für den den N-Oxidresten Y, zugrunde liegenden nicht oxidierten gesättigten stickstoffhaltigen Heterocyclus steht,

A die oben angegebene Bedeutung hat,

mit Sauerstoff abgebenden Mitteln umsetzt, oder wenn man

b) Verbindungen der Formel (IV)

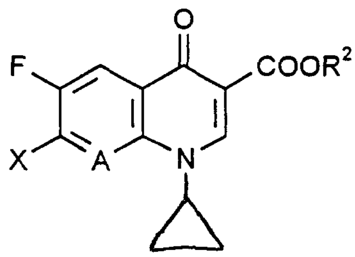

in welcher

A, $R^2$ die oben angegebene Bedeutung haben und

X Halogen, bevorzugt Fluor oder Chlor bedeutet,

mit N-Oxiden gesättigter stickstoffhaltiger Heterocyclen der Formel (V)

Y-H (V)

in welcher

Y die oben angegebene Bedeutung hat,

gegebenenfalls in Gegenwart von Säurebindern umsetzt.

Bevorzugt sind Verbindungen der Formel (I), in welcher

A für =CH- steht,

$R^1$ für $C_1$-$C_3$-Alkyl oder Cyclopropyl steht,

$R^2$ für Wasserstoff oder $C_{1-4}$-Alkyl steht,

Y für Reste der Strukturen

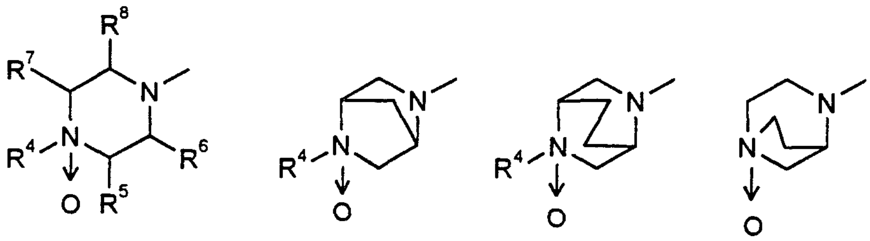

steht, worin

$R^4$ für gegebenenfalls durch Hydroxy substituiertes geradkettiges oder verzweigtes $C_1$-$C_3$-Alkyl steht,

$R^5$ für Wasserstoff, Methyl oder Phenyl steht,

$R^7$ für Wasserstoff oder Methyl steht,

und deren pharmazeutisch verwendbaren Hydrate und Säureadditionssalze sowie ihre Alkali-, Erdalkali-, Silber- und Guanidiniumsalze.

Besonders bevorzugt sind Verbindungen der Formel (I), in welcher

A für =CH- steht,

$R^1$ für Cyclopropyl steht,

$R^2$ für Wasserstoff, Methyl oder Ethyl steht,

Y für Reste der Strukturen

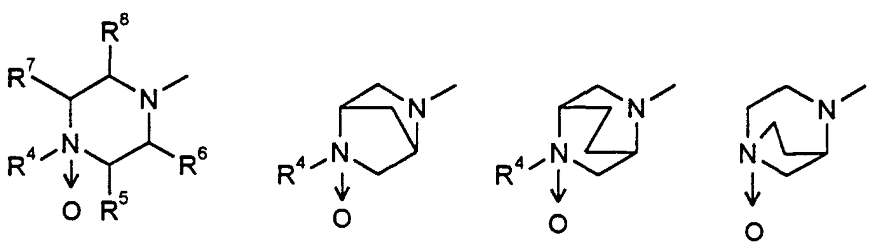

steht, worin

$R^4$ für Methyl, gegebenenfalls durch Hydroxy substituiertes Ethyl steht,

$R^5$ für Wasserstoff oder Methyl steht,

$R^7$ für Wasserstoff oder Methyl steht,

und deren pharmazeutisch verwendbaren Hydrate und Säureadditionssalze sowie die Alkali-, Erdalkali-, Silber- und Guanidiniumsalze der zugrundeliegenden Carbonsäuren.

Ganz besonders bevorzugt seien genannt die N-Oxide der Wirkstoffe mit den common names Enrofloxacin, Danofloxacin, Ofloxacin, Norfloxacin, Benofloxacin, Sarafloxacin, Difloxacin, Orbifloxacin, Marbofloxacin.

Insbesondere seien genannt die N-Oxide der Wirkstoffe Enrofloxacin, Marbofloxacin und Ofloxacin.

Verwendet man nach Methode a) zur Herstellung der neuen N-Oxide 1-Cyclopropyl-7-(4-ethyl-1-piperazinyl)-6-fluor-1,4-dihydro-4-oxo-chinolin-3 -carbonsäure und Wasserstoffperoxid als Ausgangsstoffe, so kann der Reaktionsablauf durch das folgende Formelschema wiedergegeben werden:

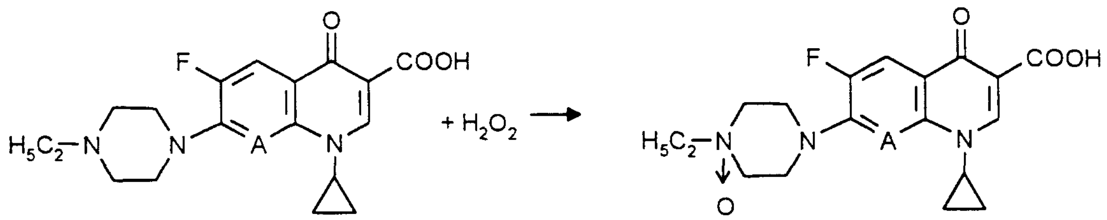

Verwendet man beispielsweise bei der Umsetzung nach Methode b) 7-Chlor-1-cyclopropyl-6-fluor-1,4-dihydro-4-oxo-chinolin-3-carbonsäureethylester und 1-Ethylpiperazin-N-oxid als Ausgangsstoffe, so kann der Reaktionsablauf durch das folgende Formelschema wiedergegeben werden:

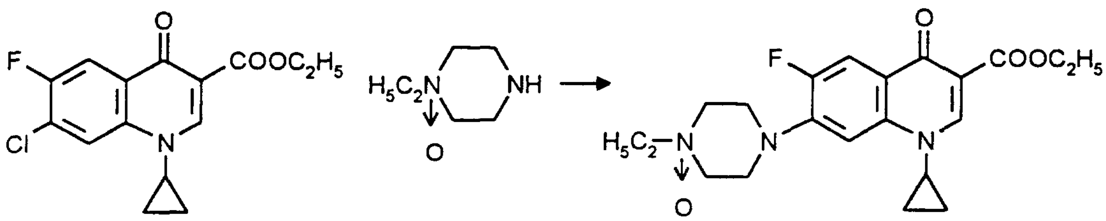

Die als Ausgangsstoffe nach Methode a) und b) verwendeten Chinoloncarbonsäuren bzw. Ester der Formeln (III) und (IV) sind bekannt oder können nach bekannten Verfahren hergestellt werden.

Als Beispiele seien genannt:
1-Ethyl-6-fluor-1,4-dihydro-4-oxo-7-(4-ethyl-1-piperazinyl)-chinolin-3-carbonsäure, 1-Ethyl-6-fluor-1,4-dihydro-7-(4-methyl-1-piperazinyl)-4-oxo-chinolin-3-carbonsäure, 1-Ethyl-6-fluor-1,4-dihydro-4-oxo-7-(4-ethyl-1-piperazinyl)-1,8-naphthyridin-3 -carbonsäure, 9-Fluor-3-methyl-10-(4-methyl-1-piperazinyl)-7-oxo-2,3-dihydro-7,4-pyrido[1,2,3-di]-1,4-benzoxazin-6-carbonsäure, 9-Fluor-5-methyl-8-(4-methyl-1-piperazinyl)-6,7-dihydro-1-oxo-1H, 5H-benzo[i,j]chinolicin-2-carbonsäure, 6-Fluor-1-(4-fluorphenyl)-1,4-dihydro-4-oxo-7-(4-methyl-1-piperazinyl)-chinolin-3-carbonsäure, 6-Fluor-1-(2-fluorethyl)-1,4-dihydro-4-oxo-7-(4-methyl-1-piperazinyl)-chinolin-3-carbonsäure, 1-Ethyl-6,8-difluor-1,4-dihydro-4-oxo-7-(4-methyl-1-piperazinyl)-chinolin-3-carbonsäure, 1-Cyclopropyl-6-fluor-1,4-dihydro-4-oxo-7-(4-ethyl-1-piperazinyl)-chinolin-3-carbonsäure, 1-Cyclopropyl-6-fluor-1,4-dihydro-4-oxo-7-(4-ethyl-1-piperazinyl)-chinolin-3-carbonsäureethylester, 1-Cyclopropyl-6,8-difluor-1,4-dihydro-4-oxo-7-(4-methyl-1-piperazinyl)-chinolin-3-carbonsäure, 1-Cyclopropyl-8-chlor-6-fluor-1,4-dihydro-4-oxo-7-(4-methyl-1-piperazinyl)-chinolin-3-carbonsäure, 1 -Cyclo-propyl-8-chlor-6-fluor-1,4-dihydro-4-oxo-7-(4-methyl-1-piperazinyl)-chinolin-3 -carbonsäure, 7-Chlor-1-ethyl-6-fluor-1,4-dihydro-4-oxo-chinolin-3-carabonsäureethylester, 1 -Ethyl-6,7-difluor-1,4-dihydro-4-oxo-chinolin-3-carbonsäureethylester, 7-Chlor-1-ethyl-6-fluor-1,4-dihydro-4-oxo-1,8-naphthyridin-3-carbonsäureethylester, 9,10-Difluor-3-methyl-7-oxo-2,3-dihydro-7,4-pyrido[1,2,3-de]-1,4-benzoxacin-6-carbonsäureethylester, 8,9-Difluor-5-methyl-6,7-dihydro-1-oxo-1H,5H-benzo[i,j]chinolicin-2-carbonsäureethylester, 7-Chlor-6-fluor-1-(4-fluorphenyl)-1,4-dihydro-4-oxo-chinolin-3-carbonsäureethylester, 6,7,8-Trifluor-1-(4-fluorphenyl)-1,4-dihydro-4-oxo-chinolin-3-carbonsäureethylester, 7-Chlor-1-cyclopropyl-6-fluor-1,4-dihydro-4-oxo-chinolin-3-carbonsäureethylester, 7-Chlor-1-cyclopropyl-6-fluor-1,4-dihydro-4-oxo-chinolin-3-carbonsäuremethylester, 7-Chlor-1-cyclopropyl-6-fluor-1,4-dihydro-4-oxo-chinolin-3-carbonsäure-n-butylester, 1-Cyclopropyl-6,7-difluor-1,4-dihydro-4-oxo-chinolin-3-carbonsäureethylester, 1-Cyclopropyl-6,7,8-trifluor-1,4-dihydro-4-oxo-chinolin-3-carabonsäureethylestser, 8-Chlor-1-cyclopropyl-6,7-difluor-1,4-dihydro-4-oxo-chinolin-3-carbonsäureethylester, 1-Cyclopropyl-7-chlor-6-fluor-1,4-dihydro-4-oxo-1,8-naphthyridin-3-carbonsäure-ethylester.

Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als weitere Beispiele seien genannt:

1-Methylpiperazin,
1-Ethylpiperazin,

N(2-Hydroxyethyl)-piperazin,
N(2-Methoxyethyl)-piperazin,
1-Cyclopropylpiperazin,
1-Phenylpiperazin,
1,2-Dimethylpiperazin,
2,5-Diazabicyclo[2.2.1]heptan,
2-Methyl-2,5-diazabicyclo[2.2.1]heptan,
2,5-Diazabicyclo[2.2.2]octan,
2-Methyl-2,5-diazabicyclo[2.2.2]octan,
1,4-Diazabicyclo[3.2.1 ]octan.

Bei der Umsetzung der Verbindungen der Formel (III) mit Sauerstoff abgebenden Mitteln werden die Verbindungen (III) als solche oder in Form ihrer Salze, wie z.B. Mesylate, eingesetzt.
Im einzelnen seien folgende Sauerstoff abgebende Mittel genannt:

Wasserstoffperoxid, Sauerstoff, organo-Hydroperoxide wie z.B. tert. Butylhydroperoxid, Cumolhydroperoxid. Dabei kann der Zusatz eines Metallkatalysators günstig sein (Mo, V, Ti).

Persäuren: wie z.B. Perameisensäure, Peressigsäure, Perbenzoesäure, Monoperphthalsäure, Sulfomonopersäure.

Peroxide wie z.B. Benzoylperoxid, Perborat, Percarbonat.

Ozon

Die Reaktionstemperaturen können in einem größeren Bereich variiert werden. Im allgemeinen arbeitet man zwischen -20°C und 200°C, vorzugsweise zwischen 20°C und 150°C.
Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.
Die Umsetzung wird vorzugsweise in einem Verdünnungsmittel vorgenommen.
Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n- und i-Propanol, Glykolmonomethylether und Wasser. Ebenso können Gemische dieser Verdünnungsmittel verwendet werden.
Die Umsetzung kann in Gegenwart von Hilfsbasen wie z.B. Alkali- und Erdalkalihydroxiden (Li, Na, K, Mg, Ca) Alkali- und Erdalkalicarbonaten und Hydrogencarbonaten (Li, Na, K,) Phosphaten, Salzen organischer Säure wie z.B. Na-Acetat durchgeführt werden.
Bei der Durchführung des erfindungsgemäßen Verfahrens setzt man auf 1 Mol der Verbindung (III) 1 bis 15 Mol, vorzugsweise 1 bis 6 Mol des Sauerstoff abgebenden Mittels ein.
Die Aufarbeitung des Reaktionsgemisches erfolgt in an sich bekannter Weise. Werden als Oxidationsmittel Persäuren verwendet, so lassen sich die entsprechenden Säureadditionssalze der Verbindungen (I) und (II) direkt gewinnen:
z.B.

Enrofloxacin + Peressigsäure $\rightarrow$ Enrofloxacin-N-oxid-acetat.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure

oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Mittel bzw. die ihnen zugrunde liegenden Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die Wirkstoffe eignen sich bei günstiger Warmblütertoxizität bevorzugt zur Bekämpfung von bakteriellen Erkrankungen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen bevorzugt enteral.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser.

Geeignete Zubereitungen sind:

Lösungen wie orale Lösungen, Konzentrate zur oralen Verabreichung nach Verdünnung;

Emulsionen und Suspension zur oralen halbfeste Zubereitungen;

Feste Zubereitungen wie Pulver, Premixe oder Konzentrate, Granulate, Pellets, Tabletten, Boli, Kapseln.

Lösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen lösen.

Als Lösungsvermittler seien genannt: Lösungsmittel, die die Lösung des Wirkstoffs im Hauptlösungsmittel

fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Oral verabreichbare Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die erfindungsgemäßen Verbindungen bzw. die daraus hergestellten oral verabreichbaren Mittel sind in den üblichen in-vitro Testsystemen zur Bestimmung minimaler Hemmkonzentrationen (MHK-Werte) antibakterieller Verbindungen praktisch unwirksam. Sie sind jedoch voll wirksam wenn sie in-vivo oral verabreicht werden.

## Beispiel 1

Herstellung von Enrofloxacin-N-oxid

20 g Enrofloxacin wurden mit 200 ml entmineralisiertem Wasser suspendiert. 25 ml $H_2O_2$ (30 %) wurden hinzugefügt und die Suspension wurde am Rückfluß gekocht. Nach 3 bis 4 Stunden war eine gelbe Lösung entstanden, aus der beim Abkühlen Kristalle ausfielen. Durch Abdampfen in einer Kristallierschale wurden weiße Kristalle erhalten. Ausbeute: 23,6 g Enrofloxacin-N-oxid roh; enthält <1 % Enrofloxacin oder andere Nebenprodukte (HPLC).

## Beispiel 2

20 g (55,7 mmol) Enrofloxacin werden in 180 ml destilliertem Wasser suspendiert und 15 ml (0,147 mol) 30%iges Wasserstoffperoxid zugegeben. Anschließend wird langsam bis zum Rückfluß erhitzt. Nach 4 Stunden wurde auf Raumtemperatur abgekühlt, abgesaugt wird im Exsiccator über Schwefelsäure getrocknet.

Ausbeute: 19,37 g (92,7%) Enrofloxacin-N-oxid
Gehalt: 99,7%
Gehalt an Enrofloxacin < 0,1%.

## Beispiel 3

2 g (5,6 mmol) Enrofloxacin werden in 20 ml Chloroform gelöst, dann 1,2 g (5,6 mmol) 80%ige 3-Chlorperbenzoesäure eingetragen. Es wird bei Raumtemperatur bis zur vollständigen Umsetzung gerührt. Der Niederschlag wird abgesaugt und getrocknet.
Ausbeute: 2,88 g (97%) Enrofloxacin-3-Chlorbenzoat.

## Beispiel 4

2 g (5,6 mmol) Enrofloxacin werden in 20 ml Acetonitril suspendiert, dann werden 0,7 ml (6,85 mmol) 30%iges Wasserstoffperoxid zugegeben. Die Mischung wird dann bis zum Rückfluß erhitzt. Nach 6 Stunden wird abgekühlt und der Niederschlag abgesaugt.

**Beispiel 5**

2 g (5,6 mmol) Enrofloxacin werden in 18 ml gesättigter Natriumhydrogencarbonatlösung suspendiert und 1,5 ml (14,7 mmol) 30%iges Wasserstoffperoxid zugegeben. Anschließend wird auf 50°C erwärmt. Nach 90 Minuten wird abgekühlt und mit Salzsäure auf pH6 gestellt. Der entstandene Niederschlag wird zwei Stunden bei Raumtemperatur nachgerührt, dann abgesaugt und getrocknet.

**Danofloxacin-N-oxid**

10 g (0,028 mol) Danofloxacin werden in 90 ml Wasser suspendiert, 7,5 ml (0,073 mol) 30 %iges Wasserstoffperoxid zugegeben und 3 Stunden zum Rückfluß erhitzt. Danach werden nochmals 7,5 ml 30 %iges Wasserstoffperoxid zugegeben und weitere 6 Stunden zum Rückfluß erhitzt. Danach wird abgekühlt, der Niederschlag abgesaugt, mit Wasser gewaschen und im Exsiccator über Schwefelsäure getrocknet.

Ausbeute: 0,71 g (79,3 % der Theorie)
Reinheit: 95,1 % (HPLC-Fläche)

Zur Reinigung wird in 150 ml Wasser heiß suspendiert, abkühlen gelassen und abgesaugt.

Ausbeute: 7,7 g (73 % der Theorie)
Reinheit: 99,5 % (HPLC-Fläche)
Gehalt an Danofloxacin : < 0,1 %
Schmelzpunkt: 256°C (Zersetzung)

**Marbofloxacin-N-oxid**

12,5 g (0,035 mol) Marbofloxacin werden in 125 ml gesättigter $NaHCO_3$-Lösung suspendiert, 9 ml (0,088 mol) 30 %iges Wasserstoffperoxid zugegeben und zwei Stunden bei 50°C gerührt. Die rötliche Lösung bleibt über Nacht bei Raumtemperatur stehen und wird danach mit konzentrierter Salzsäire auf pH 6,5 gestellt. Der ausgefallene Feststoff wird abgesaugt, mit Wasser gewaschen und im Exsiccator über Schwefelsäure getrocknet.

Ausbeute: 11,71 g (88 % der Theorie)
Reinheit: 98,5 % (HPLC-Fläche)
Gehalt an Marbofloxacin: < 0,1 %
Schmelzpunkt: 235°C (Zersetzung)

**Ofloxacin-N-oxid**

8 g (0,022 mol) Ofloxacin werden in 80 ml Wasser suspendiert, 5,9 ml (0,058 mol) 30 %iges Wasserstoffperoxid zugegeben und eine Stunde zum Rückfluß erhitzt. Danach läßt man über Nacht stehen. Der ausgefallene Feststoff wird abgesaugt, mit etwas Wasser gewaschen und im Exsiccator über Schwefelsäure getrocknet.

Ausbeute: 8,30 (quantitativ)
Reinheit: 99 % (HPLC-Fläche)
Gehalt an Ofloxacin: <0,1 %
Schmelzpunkt: 239°C (Zersetzung)

**Beispiel A**

Kälber mit je ca. 146 kg Gewicht wurden wie folgt behandelt:

a) 4 Tiere erhielten jeweils einmalig 5 mg/kg einer 10 %igen wäßrigen Injektionslösung von Enrofloxacin-Kalium-Salz intramusculär. Nach 0,5, 1, 2, 4, 6, 8, 24 Stunden p.i. wurde den Tieren Blut entnommen und der Wirkstoffspiegel im Serum bestimmt.
Von 0,5 bis 24 Stunden p.i. ließ sich die voll wirksame Dosis Enrofloxacin im Serum nachweisen.

b) 4 Tiere erhielten jeweils einmalig 5 mg/kg einer 10 %igen wäßrigen Injektionslösung von Enrofloxacin-N-oxid-Kaliumsalz intramuskulär. Nach 0,5, 1, 2, 4, 6, 8, 24 Stunden p.i. wurde den Tieren Blut entnommen und der

Wirkstoffspiegel im Serum bestimmt.
Von 0,5 bis 24 Stunden p.i. ließ sich kein Enrofloxacin im Serum nachweisen (untere Nachweisgrenze 0,01 µg/ml).

**Beispiel B**

Broiler mit je ca. 350 g Gewicht wurden wie folgt behandelt:

a) 24 Tiere erhielten jeweils einmalig 10 mg/kg einer 10 %igen wäßrigen Lösung Enrofloxacin-Kaliumsalz oral verabreicht. Nach 1, 2, 4, 6, 8, 24 Stunden wurde den Tieren Blut entnommen und der Wirkstoffgehalt im Serum bestimmt.
Von 1 bis 24 Stunden p.a. ließ sich die voll wirksame Dosis Enrofloxacin im Serum bestimmen.

b) 18 Tiere erhielten jeweils einmalig 10 mg/kg einer 10 %igen wäßrigen Lösung Enrofloxacin-N-oxid oral verabreicht. Nach 1, 2, 4, 6, 8, 24 Stunden wurde den Tieren Blut entnommen und der Wirkstoff im Serum bestimmt.
Von 1 bis 24 Stunden p.a. ließ sich die voll wirksame Dosis Enrofloxacin im Serum nachweisen.

**Patentansprüche**

**1.** Antibakterielle Mittel auf Basis von N-Oxiden gesättigter stickstoffhaltiger Heterocyclen, die durch Chinolon- oder Naphthyridoncarbonsäuren substituiert sind der Formel (I) oder (II)

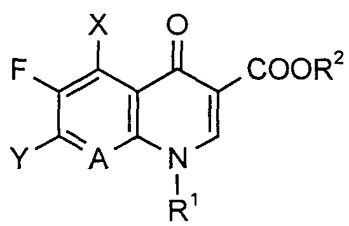

(I)

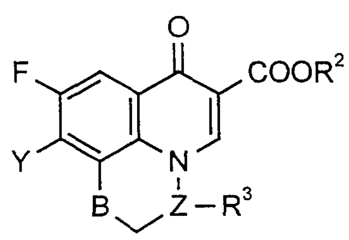

(II)

in welchen

x für Wasserstoff, Halogen, $C_{1-4}$-Alkyl, $C_{1-4}$-Alkoxy, $NH_2$ steht,

Z für =CH- oder =N- steht,

Y für Reste der Strukturen

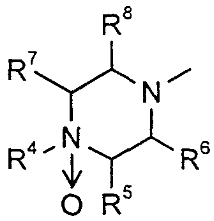
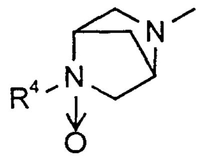
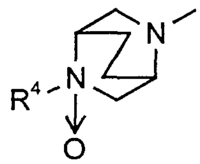
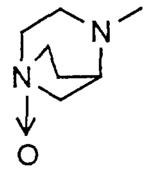

steht, worin

$R^4$ für gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

sowie

$R^1$ für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,

$R^2$ für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,

$R^3$ für Wasserstoff, Methyl oder Ethyl steht und

A für Stickstoff oder =CH-, =C(Halogen)-, =C($OCH_3$)-, =C($CH_3$)- steht,

B für Sauerstoff, gegebenenfalls durch Methyl oder Phenyl substituiertes =NH oder =$CH_2$ steht, mit Ausnahme der folgenden Verbindungen:

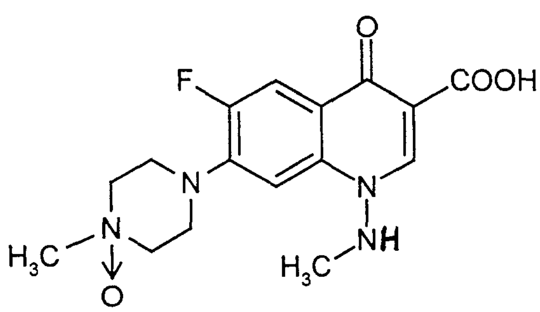

Amifloxacin-N-oxid,

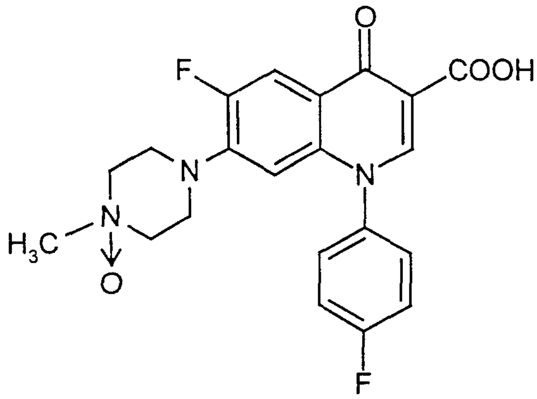

Difloxacin-N-oxid,

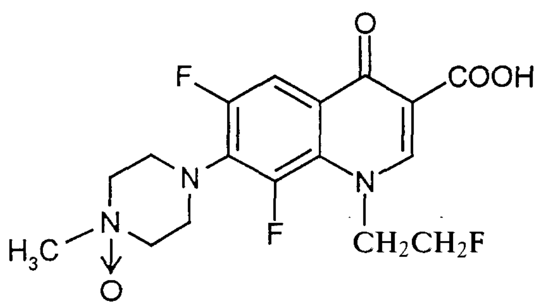

Fleroxacin-N-oxid und

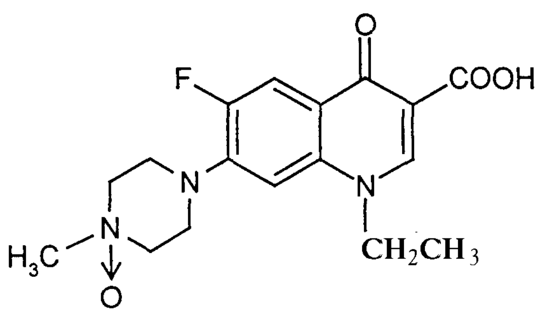

Pefloxacin-N-oxid,

und deren pharmazeutisch verwendbare Hydrate, Säureadditionssalze und Salze mit Basen.

**2.** Über das Futter und Trinkwasser von Tieren verabreichbare Mittel auf Basis von N-Oxiden gesättigter stickstoffhaltiger Heterocyclen, die durch Chinolonoder Naphthyridoncarbonsäuren substituiert sind, der Formeln (I) oder (II)

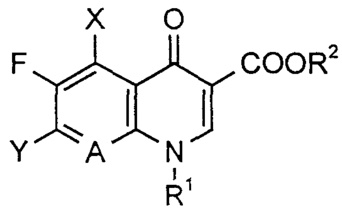

(I)

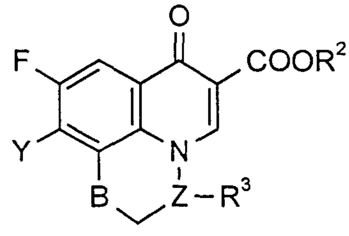

(II)

in welchen

x für Wasserstoff, Halogen, $C_{1-4}$-Alkyl, $C_{1-4}$-Alkoxy, $NH_2$ steht,

Z für =CH- oder =N- steht,

Y für Reste der Strukturen

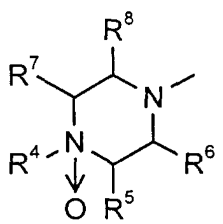 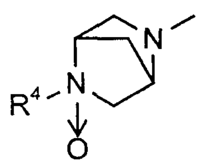 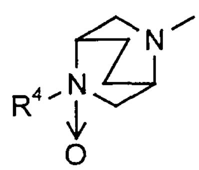 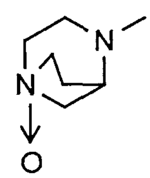

steht, worin

$R^4$ für gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

sowie

$R^1$ für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,

$R^2$ für Wasserstoff oder gegebenenfalls durch Methoxy oder 2-Methoxyethoxy substituiertes Alkyl mit 1 bis 6

Kohlenstoffatomen sowie Cyclohexyl, Benzyl, 2-Oxopropyl, Phenacyl, Ethoxycarbonylmethyl, Pivaloyloxymethyl steht,

$R^3$ für Wasserstoff, Methyl oder Ethyl steht und

A für Stickstoff oder =CH-, =C(Halogen)-, =C($OCH_3$)-, =C($CH_3$)- steht,

B für Sauerstoff, gegebenenfalls durch Methyl oder Phenyl substituiertes =NH oder =$CH_2$ steht,

mit Ausnahme der folgenden Verbindungen:

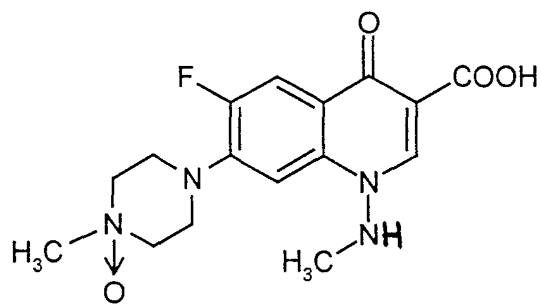

Amifloxacin-N-oxid,

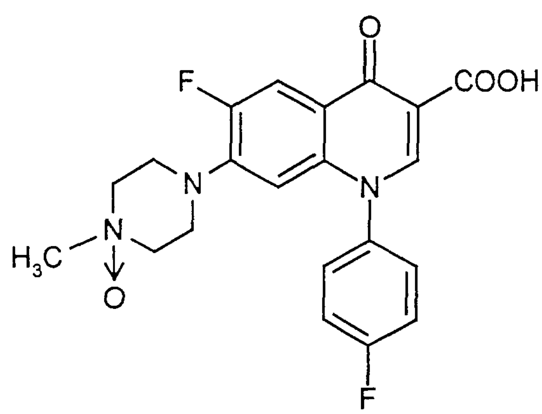

Difloxacin-N-oxid,

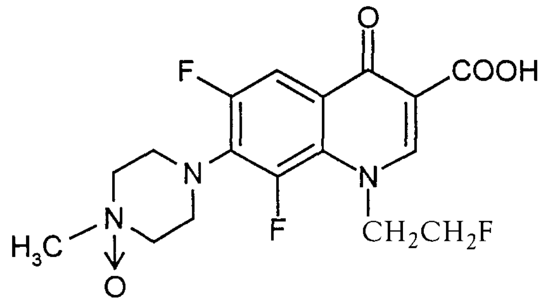

Fleroxacin-N-oxid und

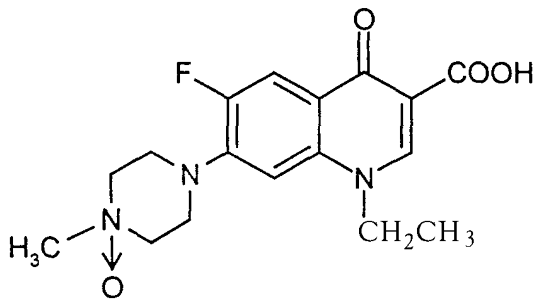

Pefloxacin-N-Oxid,

und deren pharmazeutisch verwendbare Hydrate, Säureadditionssalze und Salze mit Basen.

**3.** N-Oxide gesättigter stickstoffhaltiger Heterocyclen, die durch Chinoloncarbonsäuren substituiert sind, der Formel (Ia)

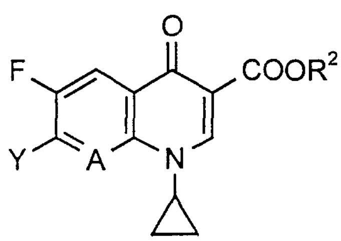

(Ia)

in welcher

Y für Reste der Strukturen

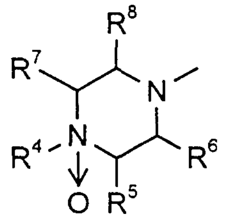
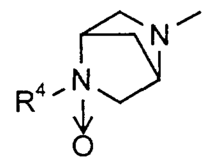
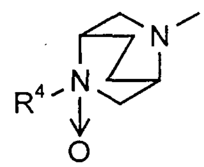
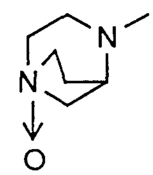

steht, worin

$R^4$ für gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

sowie

$R^2$ für Wasserstoff oder gegebenenfalls durch Methoxy substituiertes Alkyl mit 1 bis 4 Kohlenstoffatomen sowie Benzyl, 2-Oxopropyl, Phenacyl sowie Ethoxycarbonylmethyl steht,

A für =N-, =CH-, =C(Halogen)- oder =C($OCH_3$)- steht,

sowie deren pharmazeutisch verwendbare Hydrate oder Säureadditionssalze oder Salze mit Basen.

**4.** Verfahren zur Herstellung der Verbindungen der Formel (Ia) gemäß Anspruch 3, dadurch gekennzeichnet, daß man

a) die entsprechenden Verbindungen (III)

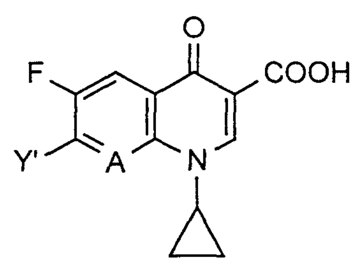

(III)

in welcher

Y' für die Reste

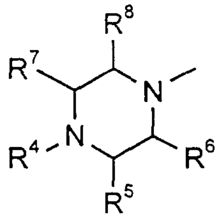 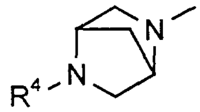 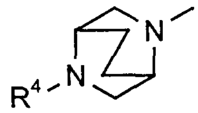 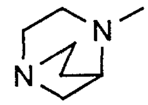

steht,
in welchen

$R^4$, $R^5$, $R^6$, $R^7$ und $R^8$ die in Anspruch 3 genannten Bedeutungen haben,

A die in Anspruch 3 angegebene Bedeutung hat,

mit Sauerstoff abgebenden Mitteln umsetzt,

oder wenn man

b) Verbindungen der Formel (IV)

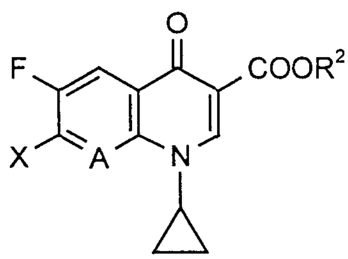

in welcher

A, $R^2$ die in Anspruch 3 angegebene Bedeutung haben und

X Halogen, bevorzugt Fluor oder Chlor bedeutet,

mit N-Oxiden gesättigter stickstoffhaltiger Heterocyclen der Formel (V)

$$\text{Y-H} \qquad \text{(V)}$$

in welcher

Y die oben angegebene Bedeutung hat,

gegebenenfalls in Gegenwart von Säurebindern umsetzt.

**5.** Verbindungen der Formel (I) gemäß Anspruch 1,
in welcher

A für=CH- steht,

$R^1$ für Cyclopropyl steht,

$R^2$ für Wasserstoff, Methyl oder Ethyl steht,

Y für Reste der Strukturen

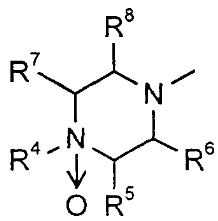
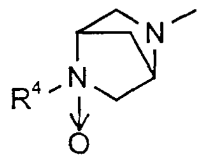
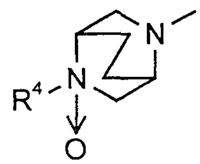
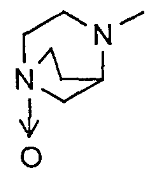

steht, worin

$R^4$ für Methyl, gegebenenfalls durch Hydroxy substituiertes Ethyl steht,

$R^5$ für Wasserstoff oder Methyl steht,

$R^7$ für Wasserstoff oder Methyl steht,

und deren pharmazeutisch verwendbaren Hydrate und Säureadditionssalze sowie die Alkali-, Erdalkali-, Silber- und Guanidiniumsalze der zugrunde liegenden Carbonsäuren.

**6.** 4-Piperazinyl-N-oxide der Formel (I) gemäß Anspruch 1 der Wirkstoffe Enrofloxacin, Marbofloxacin oder Ofloxacin.

**7.** Verwendung von Verbindungen der Formeln (I) oder (II) gemäß Anspruch 1, zur Herstellung von oral über Trinkwasser oder Futter von Tieren verabreichbaren Mitteln gegen bakterielle Erkrankungen.

**8.** Verwendung von Verbindungen der Formeln (I) oder (II) gemäß Anspruch 1 zur Herstellung von oral über das Futter von Tieren verabreichbaren Mitteln gegen bakterielle Erkrankungen.

## Claims

**1.** Antibacterial compositions based on N-oxides of saturated nitrogen-containing heterocycles which are substituted by quinolone- or naphthyridonecarboxylic acids of the formula (I) or (II)

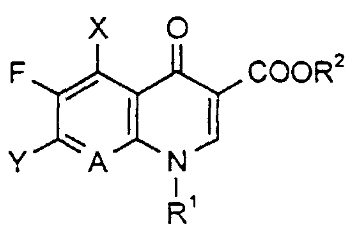

(I)

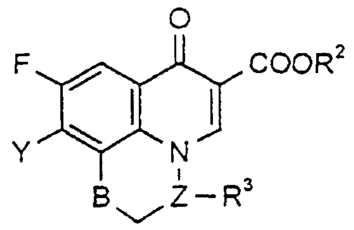

(II)

in which

X represents hydrogen, halogen, $C_{1-4}$-alkyl, $C_{1-4}$-alkoxy, $NH_2$,

Z represents = CH- or =N-,

Y represents radicals of the structures

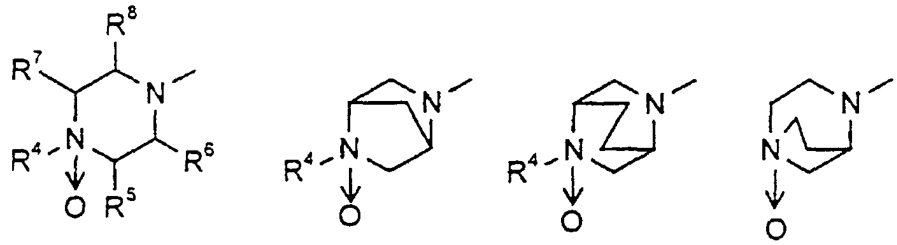

in which

$R^4$ represents straight-chain or branched $C_1$-$C_4$-alkyl which is optionally substituted by hydroxyl or methoxy, cyclopropyl, acyl having 1 to 3 C atoms,

$R^5$ represents hydrogen, methyl, phenyl, thienyl or pyridyl,

$R^6$ represents hydrogen or $C_{1-4}$-alkyl,

$R^7$ represents hydrogen or $C_{1-4}$-alkyl,

$R^8$ represents hydrogen or $C_{1-4}$-alkyl,

and

$R^1$ represents an alkyl radical having 1 to 3 carbon atoms, cyclopropyl, 2-fluoroethyl, methoxy, 4-fluorophenyl, 2,4-difluorophenyl or methylamino,

$R^2$ represents hydrogen or alkyl having 1 to 6 carbon atoms, which is optionally substituted by methoxy or 2-methoxyethoxy, and cyclohexyl, benzyl, 2-oxopropyl, phenacyl, ethoxycarbonylmethyl, pivaloyloxymethyl,

$R^3$ represents hydrogen, methyl or ethyl and

A represents nitrogen or = CH-, = C(halogen)-, = $C(OCH_3)$-, = $C(CH_3)$-,

B represents oxygen, =NH which is optionally substituted by methyl or phenyl, or = $CH_2$,

with the exception of the following compounds:

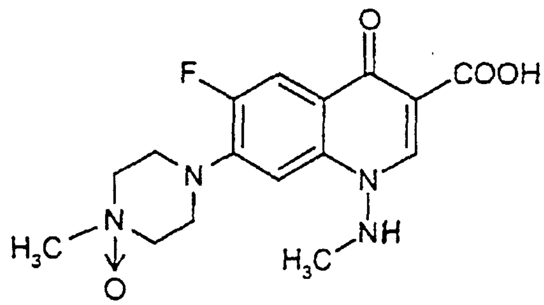

amifloxacin N-oxide,

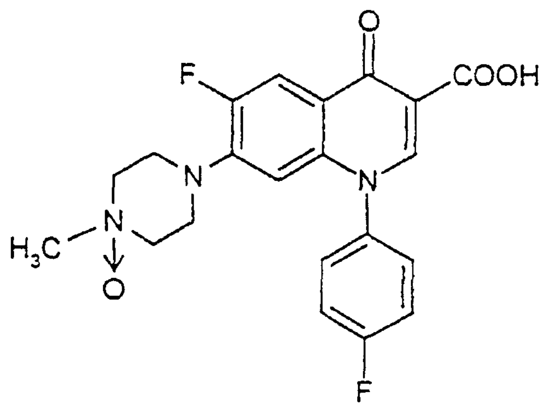

difloxacin N-oxide,

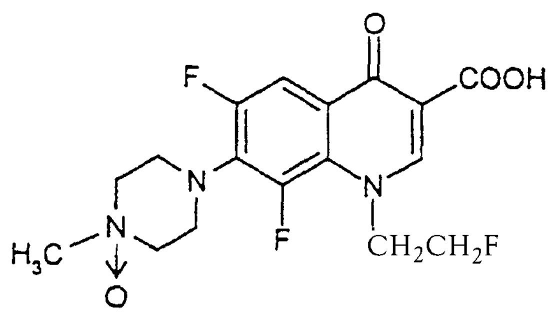

fleroxacin N-oxide and

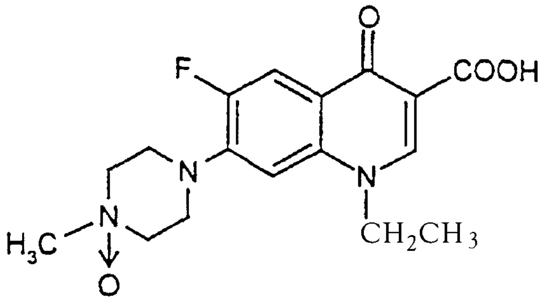

pefloxacin N-oxide,

and their pharmaceutically utilizable hydrates, acid addition salts and salts with bases.

**2.** Compositions administrable via the feed and drinking water of animals, based on N-oxides of saturated nitrogen-containing heterocycles which are substituted by quinolone- or naphthyridonecarboxylic acids, of the formula (I) or (II)

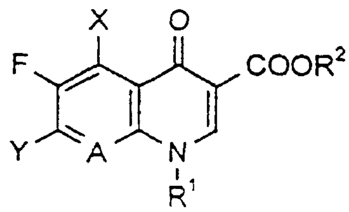

(I)

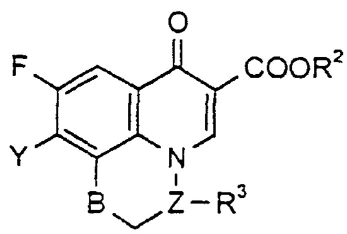

(II)

in which

X represents hydrogen, halogen, $C_{1-4}$-alkyl, $C_{1-4}$-alkoxy, $NH_2$,

Z represents = CH- or =N-,

Y represents radicals of the structures

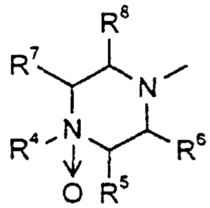 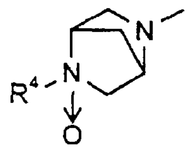 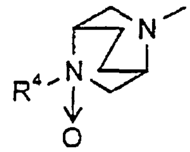 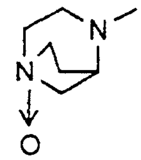

$R^4$ represents straight-chain or branched $C_1$-$C_4$-alkyl which is optionally substituted by hydroxyl or methoxy, cyclopropyl, acyl having 1 to 3 C atoms,

$R^5$ represents hydrogen, methyl, phenyl, thienyl or pyridyl,

$R^6$ represents hydrogen or $C_{1-4}$-alkyl,

$R^7$ represents hydrogen or $C_{1-4}$-alkyl,

$R^8$ represents hydrogen or $C_{1-4}$-alkyl,

and

$R^1$ represents an alkyl radical having 1 to 3 carbon atoms, cyclopropyl, 2-fluoroethyl, methoxy, 4-fluorophenyl, 2,4-difluorophenyl or methylamino,

$R^2$ represents hydrogen or alkyl having 1 to 6 carbon atoms, which is optionally substituted by methoxy or 2-methoxyethoxy, and cyclohexyl, benzyl, 2-oxopropyl, phenacyl, ethoxycarbonylmethyl, pivaloyloxymethyl,

$R^3$ represents hydrogen, methyl or ethyl and

A represents nitrogen or = CH-, = C(halogen)-, = $C(OCH_3)$, = $C(CH_3)$-,

B represents oxygen, NH which is optionally substituted by methyl or phenyl, or = $CH_2$,

with the exception of the following compounds:

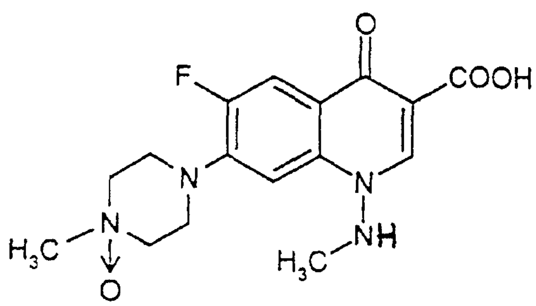

amifloxacin N-oxide,

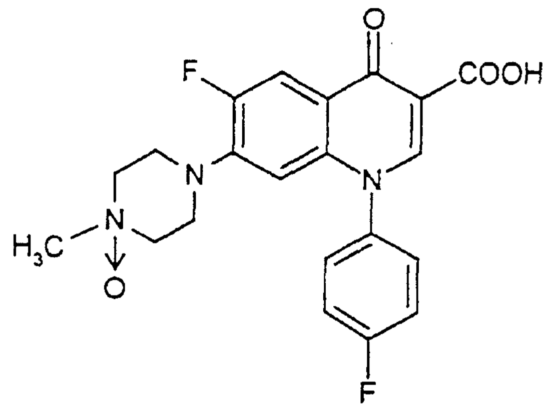

difloxacin N-oxide,

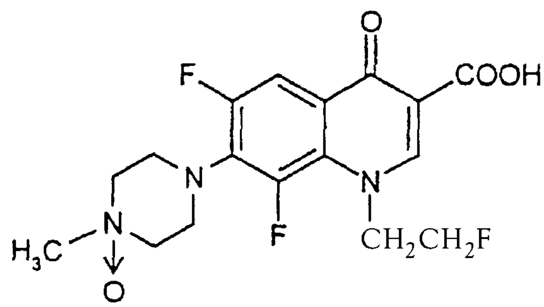

fleroxacin N-oxide and

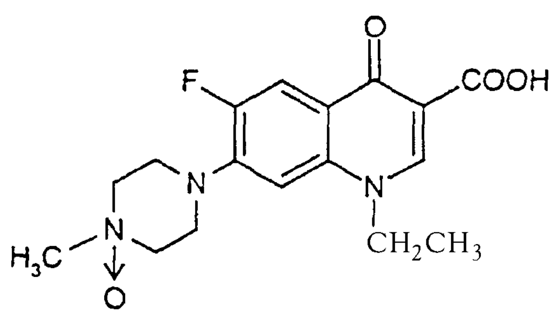

pefloxacin N-oxide,
and their pharmaceutically utilizable hydrates, acid addition salts and salts with bases.

**3.** N-Oxides of saturated nitrogen-containing heterocycles which are substituted by quinolonecarboxylic acids, of the formula (Ia)

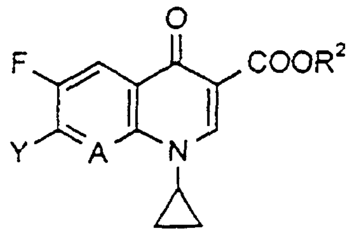

(Ia)

in which

Y represents radicals of the structures

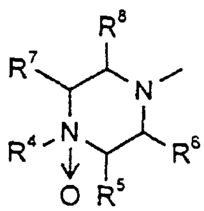 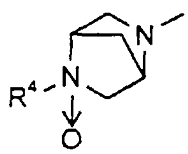 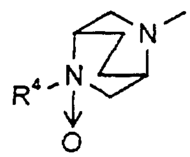 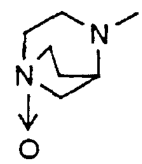

in which

$R^4$ represents straight-chain or branched $C_1$-$C_4$-alkyl which is optionally substituted by hydroxyl or methoxy, cyclopropyl, acyl having 1 to 3 C atoms,

$R^5$ represents hydrogen, methyl, phenyl, thienyl or pyridyl,

$R^6$ represents hydrogen or $C_{1-4}$-alkyl,

$R^7$ represents hydrogen or $C_{1-4}$-alkyl,

$R^8$ represents hydrogen or $C_{1-4}$-alkyl,

and

$R^2$ represents hydrogen or alkyl having 1 to 4 carbon atoms, which is optionally substituted by methoxy and benzyl, 2-oxopropyl, phenacyl and also ethoxycarbonylmethyl,

A represents =N-, = CH-, = C(halogen)- or = C($OCH_3$)-,

and their pharmaceutically utilizable hydrates or acid addition salts or salts with bases.

**4.** Process for the preparation of the compounds of the formula (Ia) according to Claim 3, characterized in that

a) the corresponding compounds (III)

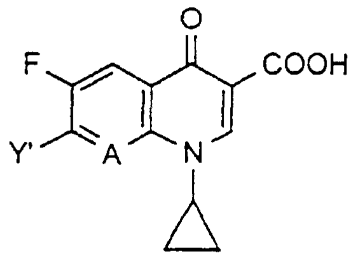

(III)

in which

Y' represents the radicals

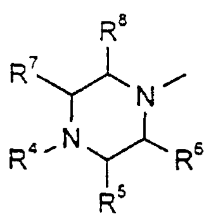 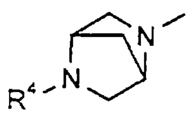 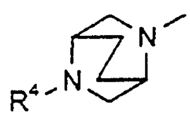 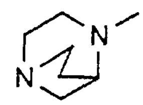

in which

$R^4$, $R^5$, $R^6$, $R^7$ and $R^8$ have the meanings mentioned in Claim 3,

A has the meaning indicated in Claim 3,

are reacted with oxygen-donating agents, or in that
b) compounds of the formula (IV)

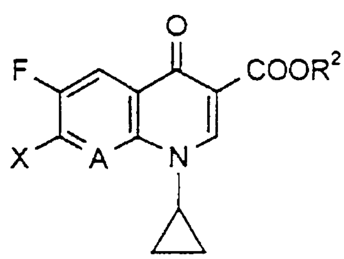

in which

A, $R^2$ have the meaning indicated in Claim 3 and

X denotes halogen, preferably fluorine or chlorine,

are reacted with N-oxides of saturated nitrogen-containing heterocycles of the formula (V)

Y - H (V)

in which

Y has the meaning indicated above,

if appropriate in the presence of acid binders.

**5.** Compounds of the formula (I) according to Claim 1, in which

A represents = CH-,

$R^1$ represents cyclopropyl,

$R^2$ represents hydrogen, methyl or ethyl

Y represents radicals of the structures

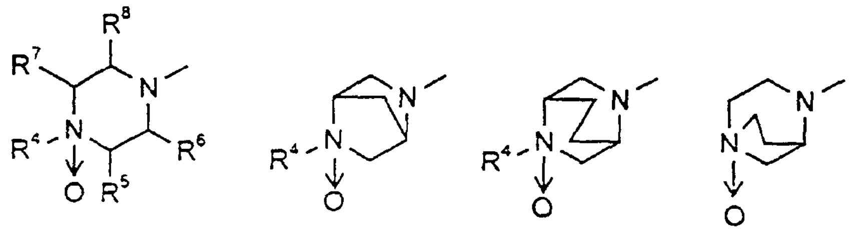

in which

$R^4$ represents methyl, ethyl which is optionally substituted by hydroxyl,

$R^5$ represents hydrogen or methyl,

$R^7$ represents hydrogen or methyl,

and their pharmaceutically utilizable hydrates and acid addition salts, and the alkali metal, alkaline earth metal, silver and guanidinium salts of the carboxylic acids on which they are based.

**6.** 4-Piperazinyl N-oxides of the formula (I) according to Claim 1 of the active compounds enrofloxacin, marbofloxacin or ofloxacin.

**7.** Use of compounds of the formula (I) or (II) according to Claim 1, for the production of compositions against bacterial disorders, which are administrable orally via drinking water or feed of animals.

**8.** Use of compounds of the formula (I) or (II) according to Claim I for the production of compositions against bacterial disorders, which are administrable orally via the feed of animals.

**Revendications**

**1.** Agents antibactériens à base de N-oxydes d'hétérocycles saturés contenant de l'azote, qui sont substitués par des acides quinolone- ou naphtyridonecarboxyliques, de formules (I) ou (II)

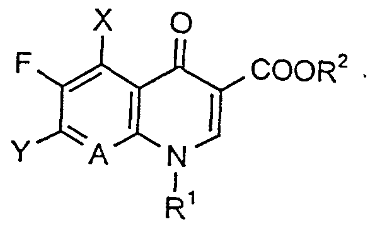

(I)

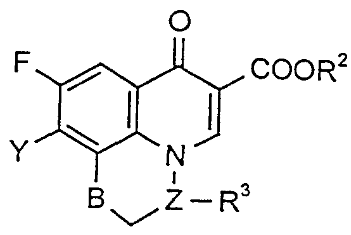

(II)

dans lesquelles

x représente l'hydrogène, un halogène, un groupe alkyle en $C_1$ à $C_4$, alkoxy en $C_1$ à $C_4$, $NH_2$,
z est un groupe =CH- ou =N-,
Y représente des restes de structures

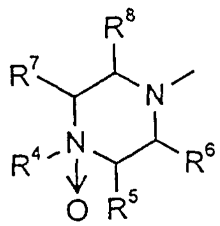
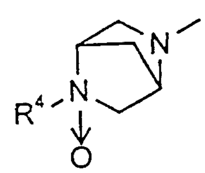
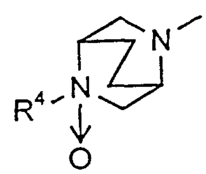
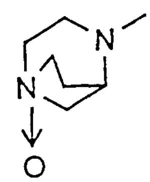

où

$R^4$ représente un groupe alkyle en $C_1$ à $C_4$ linéaire ou ramifié éventuellement substitué par un reste hydroxy ou méthoxy, un groupe cyclopropyle, un groupe acyle ayant 1 à 3 atomes de carbone,
$R^5$ représente l'hydrogène, un groupe méthyle, phényle, thiényle ou pyridyle,
$R^6$ représente l'hydrogène ou un groupe alkyle en $C_1$ à $C_4$,
$R^7$ représente l'hydrogène ou un groupe alkyle en $C_1$ à $C_4$,
$R^8$ représente l'hydrogène ou un groupe alkyle en $C_1$ à $C_4$,

de même
$R^1$ est un reste alkyle ayant 1 à 3 atomes de carbone, cyclopropyle, 2-fluoréthyle, méthoxy, 4-fluorophényle, 2,4-difluorophényle ou méthylamino,
$R^2$ représente l'hydrogène ou un groupe alkyle de 1 à 6 atomes de carbone éventuellement substitué par un reste méthoxy ou 2-méthoxy-éthoxy, de même qu'un groupe cyclohexyle, benzyle, 2-oxopropyle, phénacyle, éthoxycarbonylméthyle, pivaloyloxyméthyle,
$R^3$ représente l'hydrogène, un groupe méthyle ou éthyle et
A représente l'azote ou un groupe =CH-, =C(halogéno)-, =C($OCH_3$)-, =C($CH_3$)-,
B représente l'oxygène, un groupe =NH éventuellement substitué par un reste méthyle ou phényle ou le groupe $=CH_2$,

à l'exception des composés suivants :

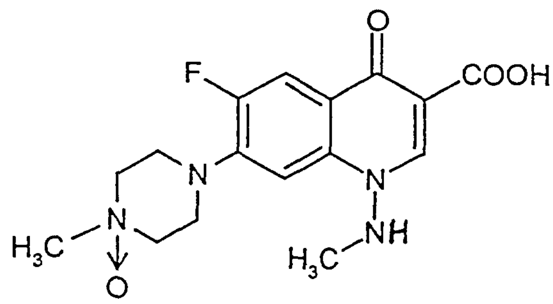

N-oxyde d'aminofloxacine,

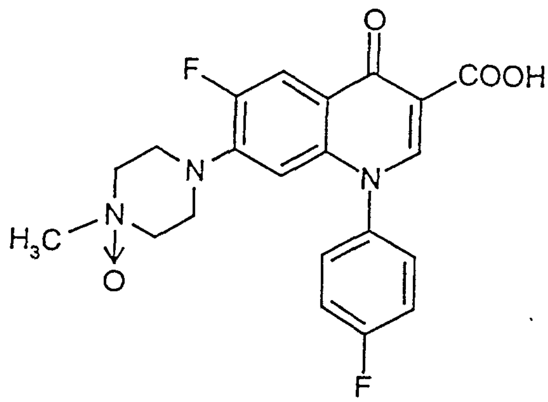

N-oxyde de difloxacine,

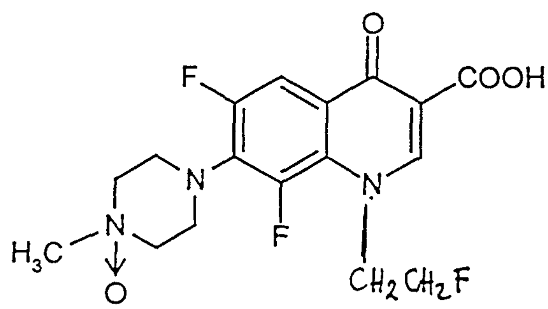

N-oxyde de fléroxacine et

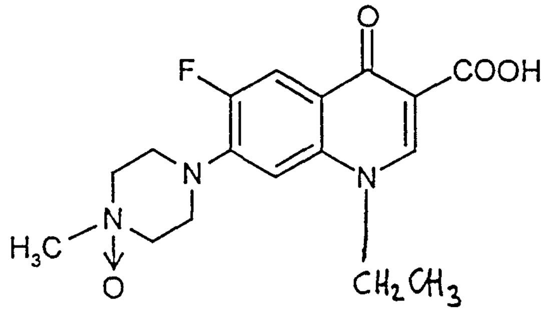

N-oxyde de péfloxacine

et leurs hydrates, leurs sels d'addition d'acides et leurs sels de bases acceptables du point de vue pharmaceutique.

**2.** Agents administrables avec la nourriture et l'eau de boisson d'animaux, à base de N-oxydes d'hétérocycles saturés contenant de l'azote, qui sont substitués par des acides quinolone- ou naphtyridone-carboxyliques, de formules (I) ou (II)

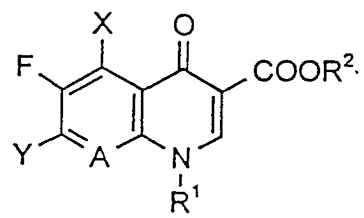

(I)

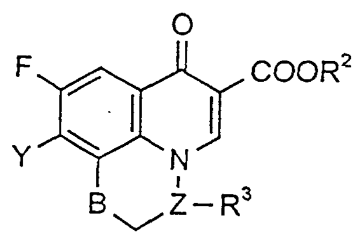

(II)

dans lesquelles

x représente l'hydrogène, un halogène, un groupe alkyle en $C_1$ à $C_4$, alkoxy en $C_1$ à $C_4$, $NH_2$,
z représente le groupe =CH- ou =N-,
Y représente des restes de structures

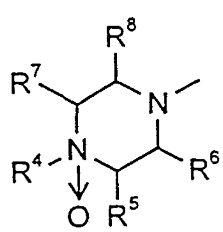
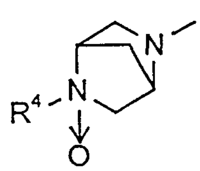
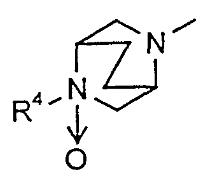
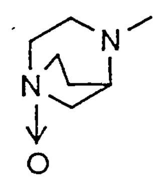

où

- $R^4$ est un groupe alkyle en $C_1$ à $C_4$ linéaire ou ramifié éventuellement substitué par un reste hydroxy ou méthoxy, un groupe cyclopropyle, un groupe acyle ayant 1 à 3 atomes de carbone,
- $R^5$ représente l'hydrogène, un groupe méthyle, phényle, thiényle ou pyridyle,
- $R^6$ représente l'hydrogène ou un groupe alkyle en $C_1$ à $C_4$,
- $R^7$ représente l'hydrogène ou un groupe alkyle en $C_1$ à $C_4$,
- $R^8$ représente l'hydrogène ou un groupe alkyle en $C_1$ à $C_4$,

de même

- $R^1$ représente un reste alkyle ayant 1 à 3 atomes de carbone, cyclopropyle, 2-fluoréthyle, méthoxy, 4-fluorophényle, 2,4-difluorophényle ou méthylamino,
- $R^2$ représente l'hydrogène ou un groupe alkyle de 1 à 6 atomes de carbone éventuellement substitué par un reste méthoxy ou 2-méthoxy-éthoxy, de même qu'un groupe cyclohexyle, benzyle, 2-oxopropyle, phénacyle, éthoxycarbonylméthyle, pivaloyloxyméthyle,
- $R^3$ représente l'hydrogène, un groupe méthyle ou éthyle,
- A représente l'azote ou un groupe =CH-, =C(halogéno)-, $=C(OCH_3)-$, $=C(CH_3)-$,
- B est l'oxygène, un groupe =NH éventuellement substitué par un reste méthyle ou phényle ou le groupe $=CH_2$,

à l'exception des composés suivants :

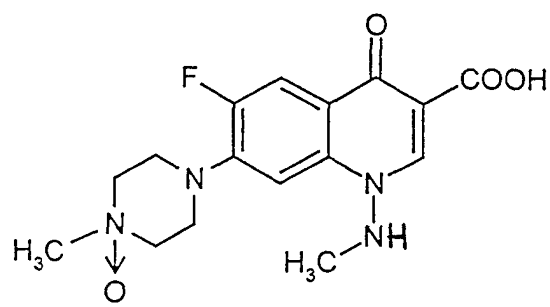

N-oxyde d'aminofloxacine,

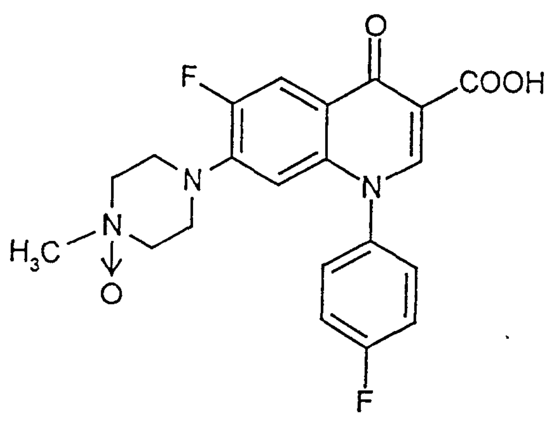

N-oxyde de difloxacine,

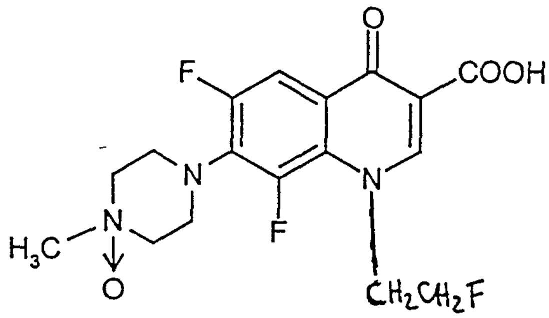

N-oxyde de fléroxacine et

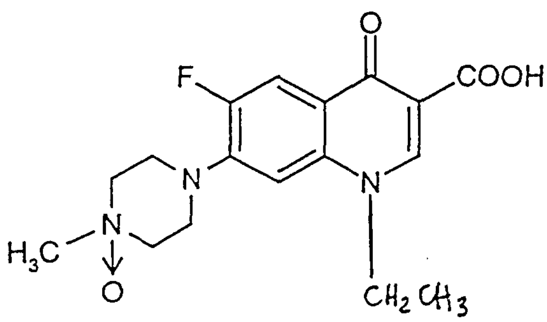

N-oxyde de péfloxacine

et leurs hydrates, leurs sels d'addition d'acides et leurs sels de bases acceptables du point de vue pharmaceutique.

**3.** N-oxydes d'hétérocycles saturés contenant de l'azote, qui sont substitués par des acides quinolonecarboxyliques, de formule (Ia)

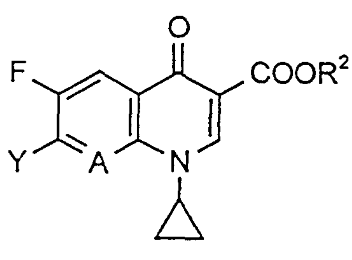

(Ia)

dans laquelle

Y représente des restes de structures

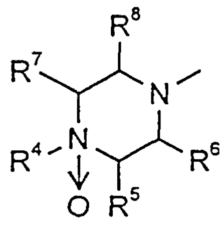
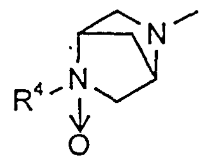
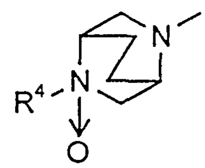
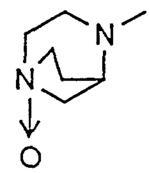

où

- $R^4$ représente un groupe alkyle en $C_1$ à $C_4$ linéaire ou ramifié éventuellement substitué par un reste hydroxy ou méthoxy, un groupe cyclopropyle, un groupe acyle ayant 1 à 3 atomes de carbone,
- $R^5$ représente l'hydrogène, un groupe méthyle, phényle, thiényle ou pyridyle,
- $R^6$ représente l'hydrogène ou un groupe alkyle en $C_1$ à $C_4$,
- $R^7$ représente l'hydrogène ou un groupe alkyle en $C_1$ à $C_4$,
- $R^8$ représente l'hydrogène ou un groupe alkyle en $C_1$ à $C_4$,

de même

- $R^2$ représente l'hydrogène ou un groupe alkyle de 1 à 4 atomes de carbone éventuellement substitué par un reste méthoxy ainsi qu'un groupe benzyle, 2-oxopropyle, phénacyle, de même qu'un groupe éthoxycarbonylméthyle,
- A représente un groupe =N-, =CH-, =C(halogéno)- ou =C($OCH_3$)-,

ainsi que leurs hydrates ou leurs sels d'addition d'acides ou leurs sels de bases acceptables du point de vue pharmaceutique.

**4.** Procédé de production des composés de formule (Ia) suivant la revendication 3, caractérisé en ce que

a) on fait réagir les composés correspondants de formule (III)

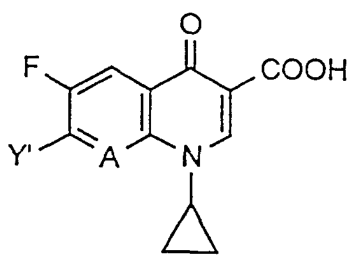

(III)

dans laquelle

Y' représente les restes

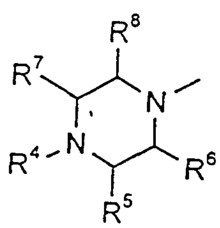
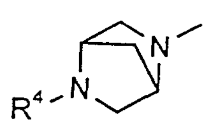
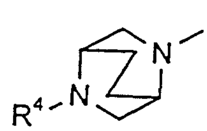
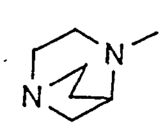

dans lesquels

$R^4$,$R^5$, $R^6$, $R^7$ et $R^8$ ont les définitions indiquées dans la revendication 3,

A a la définition indiquée dans la revendication 3,

avec des corps cédant de l'oxygène, ou bien

b) on fait réagir des composés de formule (IV)

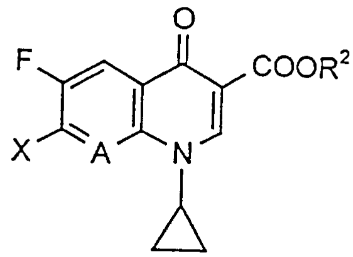

dans laquelle

A et $R^2$ ont la définition indiquée dans la revendication 3, et

X représente un halogène, de préférence le fluor ou le chlore,

avec des N-oxydes d'hétérocycles saturés contenant de l'azote, de formule (V)

$$Y - H \qquad (V)$$

dans laquelle

Y a la définition indiquée ci-dessus,

le cas échéant en présence d'accepteurs d'acides.

**5.** Composés de formule (I) suivant la revendication 1, dans laquelle

A représente le groupe =CH-,
$R^1$ représente le groupe cyclopropyle,
$R^2$ représente l'hydrogène, un groupe méthyle ou éthyle,
Y représente des restes de structures

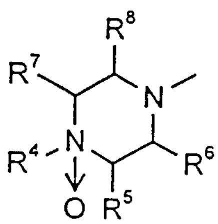 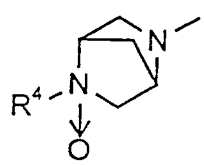 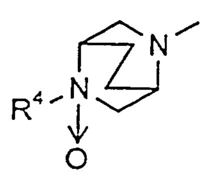 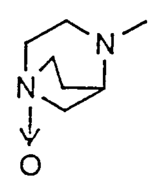

où

$R^4$ représente un groupe méthyle, un groupe éthyle éventuellement substitué par un reste hydroxy,
$R^5$ représente l'hydrogène ou le groupe méthyle,
$R^7$ représente l'hydrogène ou le groupe méthyle,

et leurs hydrates et leurs sels d'addition d'acides pharmaceutiquement acceptables ainsi que les sels de métaux alcalins, de métaux alcalino-terreux, d'argent et de guanidinium des acides carboxyliques de base.

**6.** N-oxydes de 4-pipérazinyle de formule (I) suivant la revendication 1, des substances actives enrofloxacine, marbofloxacine ou ofloxacine.

**7.** Utilisation de composés de formules (I) ou (II) suivant la revendication 1 pour la préparation d'agents contre des maladies bactériennes, administrables par voie orale par l'eau de boisson ou la nourriture d'animaux.

**8.** Utilisation de composés de formules (I) ou (II) suivant la revendication 1 pour la préparation d'agents contre des maladies bactériennes, administrables par voie orale par la nourriture d'animaux.